# EUROPEAN PATENT APPLICATION

(11) **EP 1 591 451 A1**
(43) Date of publication of application: **02.11.2005**
(21) Application number: 04708905.7
(22) Date of filing: 06.02.2004
(51) Int. Cl.: C07K 7/08, A61K 31/80, A61P 7/02, A61P 7/04, A61P 7/08

(54) **PEPTIDE CONJUGATE**

(30) Priority: 06.02.2003 JP 2003029847; 26.01.2004 JP 2004017046
(71) Applicant: Keio University, Tokyo 108-8345 (JP); Mitsubishi Pharma Corporation, Osaka-shi, Osaka 541-0046 (JP)
(72) Inventor: TAKEOKA, Shinji, Tokyo 1580094 (JP); IKEDA, Yasuo, Tokyo 1120002 (JP); HANDA, Makoto, Tokyo 1500001 (JP)
(74) Representative: von Kreisler, Alek
(86) International application number: PCT/JP2004/001293
(87) International publication number: WO 2004/069862

(57) **Abstract**

A platelet substitute which does not induce formation of unnecessary thrombi and intravascular coagulation of blood, by causing aggregation of resting platelets in the blood vessel, and has a specific aggregation action is provided. It was found as a result that a peptide conjugate, in which a synthesized form of a dodecapeptide contained in the GPIIb/IIIa recognizing site of fibrinogen is bound to a micro particle, has a property to bind specifically to only substantially activated GPIIb/IIIa, which is preferable as a platelet substitute, thereby accomplishing the present invention.

## Description

This application claims the priorities of Japanese patent application No. 2003-029847 and Japanese patent application No. 2004-017046, the entire contents of which are incorporated hereinto by reference.

### Technical Field

The present invention relates to a peptide conjugate having a platelet substitutive function. Particularly, the present invention relates to a conjugate of a micro particle with an oligopeptide capable of specifically recognizing an activated platelet, a platelet substitute which comprises the conjugate, a method for controlling platelet aggregation by using the conjugate, a process for producing the conjugate, and a diagnostic agent which comprises the conjugate.

### Background of the Invention

In the case of disease such as thrombocytopenia, or when the number of platelets is temporarily reduced in using a chemotherapeutic agent such as an antitumor agent, it causes a bleeding tendency and a symptom in which blood cannot easily be stopped in the case of bleeding. As a method for treating this symptom, human platelet preparations are administered. However, since they are derived from human, there is a problem of having an infectious risk with viruses and pathogenic bacteria, and what is more, the preservation period of the human platelet preparations is extremely short which is 3 days at room temperature, so that development of a platelet substitute which can solve these problems is in a pressing need.

Platelet preparations containing platelets collected from human are essential for the treatment of thrombocytopenia and the like, but since their preservation period is short, and also because of problems such as infectious risk, studies have been carried out on the platelet substitute which can be preserved for a long period of time.

Up to this time, a product prepared by freeze-drying human platelet fixed with paraformaldehyde is known as an older platelet substitute (cf. Patent Reference 1). This is a product in which its long-term preservation is possible by deactivating the metabolic function of platelets through the fixing treatment, while keeping back only the GPIb activity on the platelet membrane. Recently, there are known products prepared by binding functional macromolecules such as GPIb or GPIIb/IIIa, as glycoproteins which are known to be present on the platelet membrane and concerned in the adhesion or aggregation of platelets, to the surface of certain micro particles. Among these, a product having GPIb is expected to act as a platelet substitute due to the possession of adhesive action based on the interaction between GPIb and von Willebrand factor (vW factor), and on the other hand, a product having GPIIb/IIIa is expected to act as a platelet substitute due to the possession of aggregation action based on the interaction between GPIIb/IIIa and fibrinogen and/or vW factor.

Also, as the micro particles to which these functional macromolecules are bound, lipid membrane such as vesicle (liposome), human albumin or a polymer thereof, human erythrocyte and the like are known. Specifically, a product prepared by binding GPIb to vesicle (liposome) as a micro particle (cf. Patent Reference 2), a product prepared by binding GPIb to a human albumin polymer as a micro particle (cf. Patent Reference 3), a product prepared by binding GPIIb/IIIa to vesicle (liposome) as a micro particle (cf. Non-patent Reference 1) and the like are known.

In addition, although the above-described products are products which have only one kind of functional macromolecule, a platelet substitute which has both of GPIb and GPIIb/IIIa is also known. Specifically, a product in which GPIIb/IIIa on platelets is activated and then fixed with paraformaldehyde (cf. Patent Reference 4) is known.

On the other hand, unlike these products which act as a platelet substitute by possessing functional macromolecules on platelets, those which induce blood aggregation by assisting the activity of platelets remaining in the patient's blood are also known, and these can also be regarded as a platelet substitute. For example, those which induce blood aggregation by causing interaction with GPIIb/IIIa on platelets are known. Specifically, a product prepared by binding a peptide having an RGD sequence (Arg-Gly-Asp) (SEQ ID NO:2) to vesicle (liposome) (cf. Non-patent Reference 2), a product prepared by binding fibrinogen to a human albumin polymer (cf. Non-patent Reference 3), a product prepared by binding a peptide containing a dodecapeptide (His-His-Leu-Gly-Gly-Ala-Lys-Gln-Ala-Gly-Asp-Val), which is contained in the GPIIb/IIIa recognizing site of fibrinogen, to human albumin (cf. Patent Reference 5) and the like are known.

### Related References

- Patent Reference 1:: U. S. Patent No. 4,287,087
- Patent Reference 2:: JP-A-9-208599, U.S. Patent 6,177,059 and EP-A-0894807
- Patent Reference 3:: JP-A-2001-151800
- Patent Reference 4:: JP-A-2001-131078
- Patent Reference 5:: U. S. Patent No. 4,661,471
- Non-patent Reference 1:: M.E. Rybak, *Thrombosis and Haemostasis,* 55, 240-245 (1986)
- Non-patent Reference 2:: T. Nishiya and S. Sloan, *Biochim. Biophys. Res. Comun.,* 224*,* 242-245 (1996)
- Non-patent Reference 3:: S. *Takeoka et al., Biomacromolecules,* 2, 1192-1197 (2001)

### Disclosure of the Invention

One of the most important matters to take care in producing a platelet substitute is to control aggregation ability of the platelet substitute such that the produced platelet substitute does not generate serious side effects, such as vascular obstruction, by inducing formation of thrombi or intravascular coagulation of blood, caused by its aggregation in blood vessels in other than the necessary case. In fact, in human platelets, GPIIb/IIIa is resting under ordinary conditions and activated only by responding to a necessity. By this control mechanism, unnecessary formation of thrombi and coagulation of blood are inhibited in the living body. However, products equipped with such a control mechanism are not known, regarding immobilized platelets or the conventional platelet substitute in which GPIIb/IIIa is bound to a carrier.

On the other hand, a platelet substitute, which induces blood coagulation by assisting the activity of platelets remaining in the patient's blood, requires binding of fibrinogen making use of activated GPIIb/IIIa on platelets and aggregation of platelets. However, it was revealed by the studies conducted by the present inventors that a platelet substitute in which a peptide having an RGD sequence is bound to a micro particle has an action to bind even to platelets in which GPIIb/IIIa is under an resting condition, via the resting GPIIb/IIIa. This fact indicates that even a platelet substitute which assists the activity of platelets has a possibility of having side effects such as formation of unnecessary thrombi and intravascular coagulation of blood. In addition, a product prepared by binding fibrinogen to a micro particle is also expected to have a specific binding action only with activated GPIIb/IIIa, but it is suggested that there is a problem regarding unstable nature of fibrinogen (cf. the above-described Non-patent Reference 3). In addition to these, regarding the product prepared by binding a peptide containing a dodecapeptide moiety which is contained in the GPIIb/IIIa recognizing site of fibrinogen, its specific embodiments are not fully revealed in the prior art, and nothing is revealed on properties such as its stability and illustrative actions and effects (cf. the above-described Patent Reference 5).

Based on the above, there is a demand still now for a platelet substitute which does not induce formation of unnecessary thrombi and intravascular coagulation of blood, by causing aggregation of resting platelets in the blood vessel, and has a specific aggregation action.

The present inventors have conducted intensive studies on a platelet substitute having a specific aggregation action and found as a result that a peptide conjugate, in which a synthesized form of a dodecapeptide contained in the GPIIb/IIIa recognizing site of fibrinogen is bound to a micro particle, has a property to bind specifically to only substantially activated GPIIb/IIIa, which is preferable as a platelet substitute, thereby accomplishing the present invention. In addition, since human-derived components are not used in the platelet substitute of the present invention, and synthesized forms and recombinant forms of these components are used instead, the risk of causing infection with viruses and the like can be avoided.

That is, the contents of the present invention are as follows.
1. A conjugate which is a formula: 
(micro particle)-[(linker)-Cys-His-His-Leu-
Gly-Gly-Ala-Lys-Gln-Ala-Gly-Asp-Val-COOH]n,
 or a formula: 
(micro particle)-[(spacer)-(linker)-Cys-His-His-Leu-
Gly-Gly-Ala-Lys-Gln-Ala-Gly-Asp-Val-COOH]n,
 wherein n is an integer.
2. The conjugate according to the above-described item 1, wherein the micro particle is selected from the group consisting of a vesicle, a micelle, a protein polymer and a synthetic polymer.
3. The conjugate according to the above-described item 1 or 2, wherein the micro particle is selected from the group consisting of a vesicle, a recombinant albumin polymer, a latex particle and a biodegradable polymer.
4. The conjugate according to any one of the above-described items 1 to 3, wherein the micro particle has a particle diameter of 50 nm or more.
5. The conjugate according to any one of the above-described items 1 to 4, wherein the micro particle has a particle diameter of 10 µm or less.
6. The conjugate according to any one of the above-described items 1 to 5, wherein the linker is a compound in which its one terminal reacts with an SH group, and another terminal reacts with any one of an OH group, a COOH group and an NH₂ group.
7. The conjugate according to any one of the above-described items 1 to 6, wherein the linker is selected from the group consisting of dicarboxylic acid, aminocarboxylic acid, a bismaleimide compound, a bishalocarbonyl compound, a halocarbonylmaleimide compound, dithiomaleimide, dithiocarboxylic acid and maleimidecarboxylic acid.
8. The conjugate according to any one of the above-described items 1 to 7, wherein the linker is selected from the group consisting of dicarboxylic acid, aminocarboxylic acid, a bismaleimide compound, a bishalocarbonyl compound, a halocarbonylmaleimide compound, dithiomaleimide, dithiocarboxylic acid and maleimidecarboxylic acid, and has a carbon chain of C2-10.
9. The conjugate according to any one of the above-described items 1 to 8, wherein the spacer is one or at least two combination selected from the group consisting of polyoxyethylene, a polypeptide, a polysaccharide, albumin and an antibody.
10. The conjugate according to any one of the above-described items 1 to 9, wherein the micro particle is a latex particle, the spacer is albumin and the linker is dithiocarboxylic acid.
11. The conjugate according to any one of the above-described items 1 to 9, wherein the micro particle is a vesicle, the spacer is polyethylene glycol and the linker is maleimidecarboxylic acid.
12. A platelet substitute which comprises the conjugate according to any one of the above-described items 1 to 11.
13. A method for controlling platelet aggregation, which comprises using the conjugate according to any one of the above-described items 1 to 11.
14. A process for producing the conjugate according to any one of the above-described items 1 to 11.
15. A diagnostic agent or reagent which comprises the conjugate according to any one of the above-described items 1 to 11.
16. The diagnostic agent or reagent according to the above-described item 15, which is a diagnostic agent for platelet dysfunction, a biological or medical reagent, a platelet aggregation inhibitor, a reagent for screening an anti-thrombus agent, or a diagnostic or therapeutic agent for inspecting a vascular injury region and a thrombus formation region.
17. A drug carrier which comprises the conjugate according to any one of the above-described items 1 to 11.
18. The drug carrier which comprises the conjugate according to the above-described item 17, wherein the drug is selected from the group consisting of a hemostatic agent, a vasoconstrictor agent, an anti-inflammatory agent, an anti-blood coagulation agent and an anti-platelet agent.
19. A pharmaceutical composition which comprises the conjugate according to any one of the above-described items 1 to 11.
20. The pharmaceutical composition which comprises the conjugate according to the above-described item 19, which comprises a drug selected from the group consisting of a hemostatic agent, a vasoconstrictor agent, an anti-inflammatory agent, an anti-blood coagulation agent and an anti-platelet agent.
21. The pharmaceutical composition according to the above-described item 19 or 20, which is useful for preventing or treating vascular disorder, vascular injury and thrombosis.

### Brief Description of the Drawings

Fig. 1 shows periodical change in the surface coverage of platelets on collagen surface.
Fig. 2 shows periodical change in the surface coverage of platelets on dodecapeptide-coupled latex beads (H12-LB).
Fig. 3 shows SEM observation illustration of surface after flow
Fig. 4 shows an aggregation ratio as a bindability test to resting platelets.
Fig. 5 shows an introduced amount of H12 into vesicles and platelet aggregation effect.
Fig. 6 shows a surface coverage of platelets on collagen surface.

### Best Mode for Carrying Out the Invention

The peptide conjugate of the present invention is a formula: 
(micro particle)-[(linker)-Cys-His-His-Leu-
Gly-Gly-Ala-Lys-Gln-Ala-Gly-Asp-Val-COOH]n,
 or a formula: 
(micro particle)-[(spacer)-(linker)-Cys-His-His-Leu-
Gly-Gly-Ala-Lys-Gln-Ala-Gly-Asp-Val-COOH]n.
 In the formula, n is an integer. The dodecapeptide "His-His-Leu-Gly-Gly-Ala-Lys-Gln-Ala-Gly-Asp-Val" is a conventionally known amino acid sequence (SEQ ID NO:1) contained in the GPIIb/IIIa recognizing site of fibrinogen. This sequence can be synthesized by a conventionally known synthetic means and is not particularly limited (*The Peptides Analysis, Synthesis, and Biology,* Vol. 2, pp. 1-284, Academic Press (1980)). The terminal carboxyl group is an amino acid Val origin.

In the formula, the micro particle means a carrier for medical use which can be parenterally administered, and is not particularly limited so long as it has biocompatibility. As the suitable micro particle materials conventionally known so far, a vesicle, a micelle, a protein polymer, a synthetic polymer and the like are known, and specific examples thereof include a vesicle (liposome), a recombinant albumin polymer, a latex particle, a biodegradable polymer and the like.

The vesicle (liposome) is a particle constituted by an artificial lipid membrane, which is produced as a lipid bilayer from a phospholipid, a glyceroglycolipid, cholesterol and the like. For its preparation, a broad range of conventionally known methods, such as a surfactant removing method, a hydration method, an ultrasonic wave method, a reverse phase distillation method, a freeze-thawing method, an ethanol injection method, an extrusion method and a high pressure emulsifying method, can be employed. Details of the preparation of the vesicle (liposome) are minutely described the above-described Patent Reference 2 and Non-patent Reference 2. Although the vesicle (liposome) was used in the inventive examples and test examples of the present invention, those which have biocompatibility can be optionally selected and used.

As the recombinant albumin, those which are produced by already known genetic engineering techniques can be used with no particular limitation. For example, a recombinant albumin produced using yeast as a host at a materializing level is suitable. Micro particle formation (polymerization) of albumin is conventionally known, and the albumin polymer preparation method disclosed by the present inventors in JP-A-2001-151800 is suitable. In addition, the method for preparing recombinant albumin is minutely described in the following methods (each of JP-A-5-317079, JP-A-6-56883, JP-A-6-245789 and JP-A-8-116985), and the rHSA to be used in Example 1 was obtained by these methods.

The latex particle was used in Examples of the present invention, and those which have biocompatibility are optionally selected and used. The biodegradable polymer is prepared, for example, by making a polymer obtained by polymerizing lactic acid and/or glycolic acid into micro particles. They are used by determining molecular weights, copolymer compositions and mixing ratio such that they have periodically degrading or dissolving property in the living body.

The particle diameter of the micro particles prepared in this manner is preferably about 50 nm or more, and preferably about 10 µm or less, in view of the introducing amount of the surface recognizing region and its function exertion and intracorporeal kinetics. It is further preferably from about 50 nm to about 500 nm, and more preferably from about 100 nm to about 400 nm.

The linker in the formula is not particularly limited, so long as it can be crosslinked between the micro particle and an SH group at the peptide terminal and is a conventionally known biologically acceptable compound, but is preferably a compound in which its one terminal reacts with an SH group, and the other terminal reacts with any one of an OH group, a COOH group and an NH₂ group. Examples of the linker include dicarboxylic acid, aminocarboxylic acid, a bismaleimide compound, a bishalocarbonyl compound, a halocarbonylmaleimide compound, dithiomaleimide, dithiocarboxylic acid, maleimidecarboxylic acid and the like. It is preferable that the carbon chain thereof is C2-10.

The spacer in the formula is not particularly limited, so long as it is present between the micro particle and the linker, can adjust the length between the micro particle surface and the peptide and has biocompatibility. A substance selected from polyoxyethylene, a polypeptide, a polysaccharide, albumin and an antibody or a combination of two or more thereof can be used.

It is preferable that the number of molecules of the dodecapeptide to be bound on the micro particle is high density, because probability of binding with platelets is increased and formation of aggregate quickly progresses. In the formula, n means an integer and represents the number of dodecapeptide molecules bound to the micro particle. Such a number can be optionally changed and adjusted by those skilled in the art in response to the desired aggregation degree and aggregation rate. For example, when a vesicle (liposome) is used as shown in Test Example 4, the optimum value of the modification ratio by dodecapeptide (e.g., from 0.3 mol % to 0.6 mol %) can be specified, and its optimization can be attained by changing the conditions in the same manner. The effect becomes insufficient at a certain value or less of the modification ratio, and a certain value or more of the modification ratio does not have influence upon the effect.

After preparation of a micro particle, a conjugate of the micro particle, a linker or spacer-linker and a dodecapeptide is prepared by making the micro particle surface into such a state that a chemical binding is carried out, through its activation if necessary, subsequently allowing a necessary linker or spacer-linker to bind thereto, and further allowing a dodecapeptide to react therewith. Alternatively, a reaction product of a linker or spacer-linker and a dodecapeptide may be prepared in advance, which is finally allowed to react with the activated micro particle. As the reaction conditions, any conventionally known method suited for respective micro particle can be employed. The mixing ratio of the dodecapeptide and the micro particle is adjusted based on the desired value of the density of dodecapeptide in the final conjugate.

In the case of a vesicle, the vesicle surface can be modified with a dodecapeptide by mixing an amphipathic molecule linked by the dodecapeptide which can become a constituting component of a lipid bilayer in advance with lipids which constitute the vesicle in an organic solvent, and then preparing the vesicle in the usual way.

Next, if necessary, the conjugate of the present invention is washed with a physiologically acceptable aqueous solution, subjected to sterile filtration, dispensed and then prepared as liquid preparations, pellets and suspension preparations. Pharmaceutical preparations are prepared in accordance with the broadly and conventionally known methods in the production methods of medicaments. In addition, the pharmaceutical preparations can also be provided as freeze-dried preparations by freezing liquid preparations and then drying them under a reduced pressure. In this connection, when freeze drying is carried out, a monosaccharide (e.g., glucose, *etc.),* a disaccharide (e.g., sucrose, *etc.)* or the like may be formulated as a protecting agent. The pharmaceutical preparations of the present invention may be formulated with a macromolecular substance as a stabilizing agent selected from albumin, dextran, a vinyl polymer, gelatin and hydroxyethyl starch. The amount of the stabilizing agent to be added is from 0.5 to 10 parts by weight, preferably from 1 to 5 parts by weight, based on 1 part by weight of lipid.

Since the thus prepared pharmaceutical preparations containing the conjugate of the present invention show selective binding with activated platelet GPIIb/IIIa, they do not cause aggregation with resting platelets in blood vessels, and their aggregation with platelets at a platelet-activated region such as a vascular wound region, so that a means for controlling platelet aggregation is provided. The conjugate of the present invention can by itself become a substitute for a synthetic platelet. In addition, since the conjugate of the present invention has a property to accumulate into a platelet-activated region such as a vascular wound region, it can take an embodiment as a drug-containing composition (drug carrier). Such a drug is not particularly limited, so long as it is physiologically and pharmacologically effective when accumulated into a vascular wound region, and examples thereof include a hemostatic agent, a vasoconstrictor agent, an anti-inflammatory agent, an anti-blood coagulation agent, an anti-platelet agent and the like. The conjugate of the present invention can be administered at a dose of approximately from 0.001 to 1,000 mg as the dodecapeptide. The dose can be optionally increased or decreased in response to the sex, age, symptoms and the like of each patient. More desirably, the conjugate of the present invention is parenterally administered. Specific examples include intravascular (intraarterial or intravenous) injection, continuous drip infusion, subcutaneous administration, topical administration, intramuscular administration and the like. Examples of the pharmaceutical composition containing the conjugate of the present invention include medicaments such as a platelet substitute, a preventive or therapeutic agent for vascular disorder, vascular wound and thrombosis, or a diagnostic agent for platelet dysfunction such as thrombasthenia, a biological or medical reagent, a reagent for screening a platelet aggregation inhibitor or anti-thrombus agent, and the like. It is also useful as a diagnostic agent for inspecting a vascular wound region and thrombus formation region or a therapeutic agent for the same.

### Examples

The present invention is described below in detail with reference to production examples and test examples, although the present invention is not limited thereto.

### Example 1

### Method for synthesizing dodecapeptide [hereinafter referred to as "H12" (SEQ ID NO:1)]-coupled latex beads (hereinafter referred to as "H12-LB")

A recombinant human serum albumin (hereinafter referred to as "rHSA") solution (50 mg/ml, 1.5 ml) was mixed with a dispersion of latex beads (hereinafter referred to as "LB") of 1 µm in particle diameter and shaken (20°C, 2 hr). LB was precipitated by centrifugation (13000 g, 5 min, 4°C, 3 times) to remove un-adsorbed rHSA as supernatant, and then re-dispersed in phosphate-buffered saline (hereinafter referred to as "PBS", 500 µl).

An SPDP (N-succinimidyl 3,2-pyridyldithiopropionate) ethanol solution (5 mM, 5 µl) was added to the HSA-adsorbed LB dispersion [hereinafter referred to as "(HSA)LB"] (4.0×10⁶ particles/µl, 500 µl), followed by shaking (20°C, 30 min). By removing unreacted SPDP and by-products through centrifugation (13000 g, 5 min, 4°C, 3 times), pyridyl disulfide (hereinafter referred to as" PD")-coupled (HSA)LB [hereinafter referred to as "PD-(HSA)LB"] was obtained. This PD-(HSA)LB (4.0×10⁶ particles/µl, 500 µl) was mixed with Cys-H12 (H12 in which Cys was added to its terminal) (10 mM, 8 µl), followed by shaking (20°C, 12 hr). After removing the supernatant by centrifugation (13000 g, 5 min, 4°C, 3 times), H12-LB (2.0×10⁶ particles/µl, 1 ml) was obtained. The coupled amount of H12 on the LB surface was determined from the absorbance at 343 nm of 2-thiopyridone (hereinafter referred to as "2TP") formed by the thiol-disulfide exchange reaction (HPLC, TSK-GEL G3000SWXL column, 7.8 mm o.d. × 300 mm h, 1 ml/min, PBS).

### Example 2

### Method for synthesizing H12 (SEQ ID NO:1)-coupled vesicle (hereinafter referred to as "H12-PEG vesicle"):

PEG-Glu2C18 was synthesized as a control of H12-PEG-Glu2C18. Glutamic acid (2.96 g, 20 mmol) and p-toluenesulfonic acid monohydrate (4.56 g, 24 mmol) were dissolved in 150 ml of benzene, followed by refluxing at 105°C for 1 hour while removing the formed water using Dean-Stark apparatus. Stearyl alcohol (11.9 mg, 44 mmol) was added thereto, followed by refluxing at 105°C for 14 hours while removing the formed water. The solvent was evaporated under a reduced pressure, and then the residue was dissolved in 150 ml of chloroform and washed twice with 150 ml of a saturated aqueous sodium carbonate solution and twice with 150 ml of water. The chloroform layer was collected and dehydrated using 5 g of sodium sulfate, and then the solvent was removed under a reduced pressure. The residue was dissolved in 400 ml of methanol at 60°C, and after filtering insoluble matter when present, recrystallized at 4°C, filtered and then dried to obtain Glu2C18 (13.3 g, yield 85%) (cf. formula 1) as a white powder.

This Glu2C18 (50.08 mg, 76.8 µmol) and MeO-PEG-COOH (α-carboxyl-ω-methoxy polyethylene glycol) (Mw = 5150) (200 mg, 38.4 µmol) and HOBT (1-hydroxy-benzotriazole) (5.2 mg, 38.4 µmol) were dissolved in 5 ml of dichloromethane, and then DCC (N,N'-dicyclohexylcarbodiimide) (9.58 mg, 46.4 µmol) was added thereto, followed by stirring for 12 hours. After removing the insoluble matter by filtration, the reaction solution was added dropwise to 250 ml of diethyl ether, and the insoluble matter was collected. The collected matter was dissolved in benzene, followed by freeze drying to obtain PEG-Glu2C18 (204 mg, yield 83%) (n = 115) (cf. formula 1) as a white powder. An outline of the synthesis is shown in formula 1.

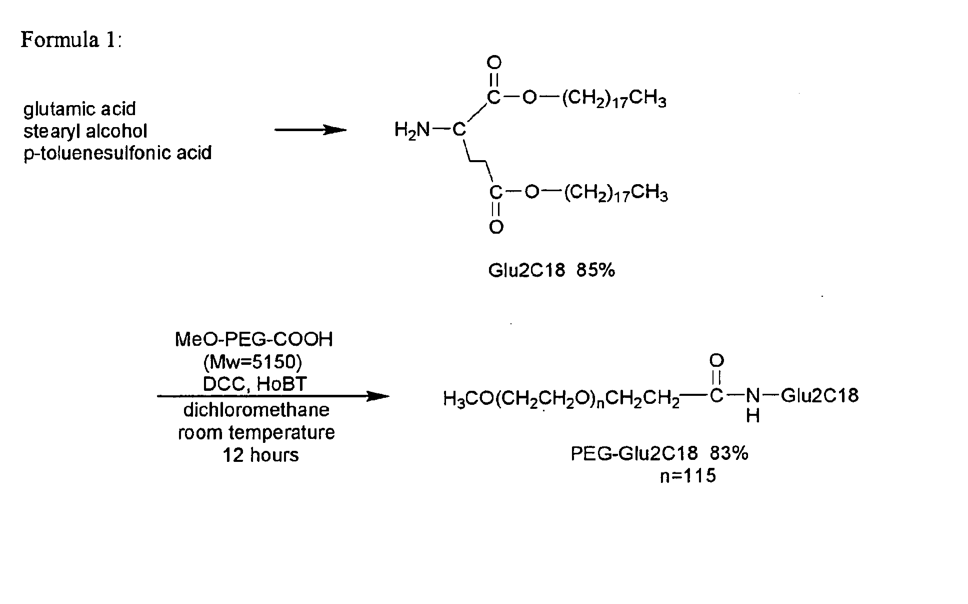

On the other hand, the H12-PEG-Glu2C18 of the present invention was prepared in the following manner. Glu2C18 (115.1 mg, 176 µmol) and TEA (triethylamine) (24.6 µl, 176 µmol) were added to 5 ml of chloroform, and then MAL-PEG-NHS (α-maleimidyl-ω-N-hydroxysuccinimidyl polyethylene glycol) (Mw = 3400) (300 mg, 58.8 µmol) was dissolved therein, followed by stirring at room temperature for 12 hours. The reaction solution was added dropwise to 250 ml of diethyl ether, and the insoluble matter was collected. The collected matter was dissolved in benzene, followed by freeze drying to obtain MAL-PEG-Glu2C18 (264.8 mg, yield 64%) (cf. formula 2) as a white powder. This MAL-PEG-Glu2C18 (n = 71) (100 mg, 25.37 µmol) and Cys-H12 (32.8 mg, 25.37 µmol) were dissolved in 5 ml of DMF, followed by stirring at room temperature for 12 hours. The reaction solution was added dropwise to 250 ml of diethyl ether, and the insoluble matter was collected. After adding water and removing the insoluble matter, the solvent was removed by a freeze dryer to obtain H12-PEG-Glu2C18 (62.8 mg, yield 47%) (cf. formula 2) as a pale yellow powder. An outline of the synthesis is shown in formula 2.

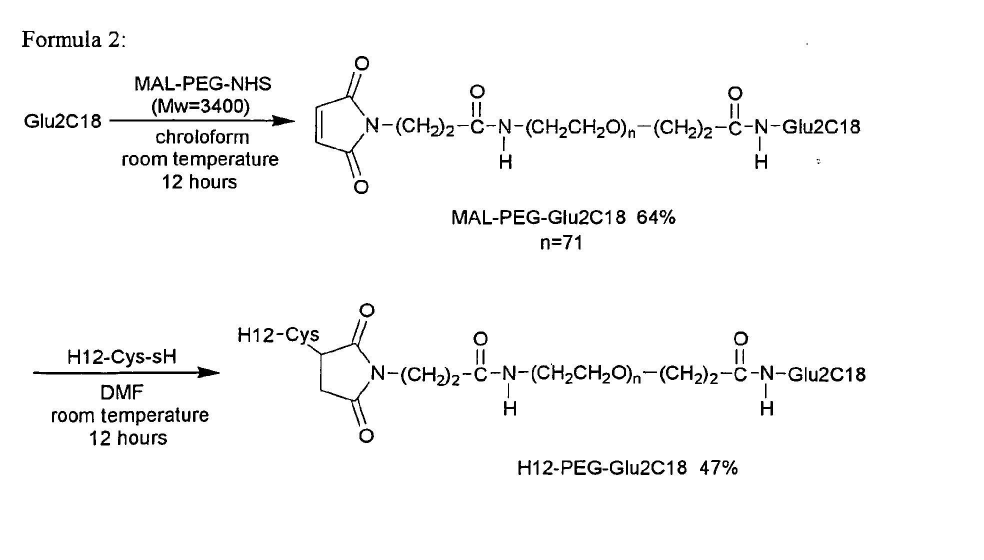

Next, H12 was introduced into vesicles using the PEG-Glu2C18 and H12-PEG-Glu2C18 prepared in the above. A mixed lipid prepared by adding PEG-Glu2C18 and/or H12-PEG-Glu2C18 at an optional ratio to DPPC (100 mg, 136 µmol) and cholesterol (52.7 mg, 136 µmol) was dissolved in 5 ml of benzene, followed by freeze drying for 3 hours. After the drying, 10 ml of purified water was added thereto, followed by stirring for 12 hours, and then particle diameter control was carried out by passing through filters having pores using a granulation device (extruder). The mixture was passed through filters in order of {3000 nm → 800 nm → 650 nm → 450 nm → 300 nm → 220 nm (x 2)}. By carrying out centrifugation (33000 rpm, 30 minutes) twice, the vesicles were dispersed in 5 ml of PBS to obtain a vesicle dispersion. Regarding the contained amounts of PEG-Glu2C18 and/or H12-PEG-Glu2C18, a vesicle containing PEG-Glu2C18 alone (4.74 mg, 0.817 µmol) was defined as PEG-vesicle, and a vesicle containing H12-PEG-Glu2C18 alone (4.34 mg, 0.817 µmol) as H12-PEG-vesicle, and a vesicle containing PEG-Glu2C18 (4.74 mg, 0.817 µmol) and H12-PEG-Glu2C18 (14.34 - 0.34 mg, 2.72 - 0.0817 µmol) as H12-PEG(PEG) vesicle.

In this connection, the prepared phospholipid vesicles (H12-PEG vesicles, 1.8 wt %) were diluted 30 times with PBS and shaken at 37°C for 12 hours, and then the vesicles were precipitated by centrifugation (33000 rpm, 1 h) and dispersed, followed by freeze drying, and the resulting powder was subjected to ¹H-NMR measurement. Since the molar ratio of DPPC, cholesterol and H12-PEG-Glu2C18 did not change when the H12-PEG vesicles were shaken at 37°C for 12 hours, it was confirmed that the H12-PEG-Glu2C18 was not released from the phospholipid vesicles but stably maintained therein.

### Test Example 1

### Aggregation test with platelets when latex beads are used as the carrier:

A thrombocytopenia model blood was prepared by passing whole blood through a leukocyte removing filter for leukocyte depleted preparations (NEOIJ, Nihon Pall) by gravity, and adjusting the platelet concentration to 2.0×10⁴/µl by adding platelet rich plasma (PRP) (an automatic blood cell counter K-4500, Sysmex, Kobe).

H12-LB (1.0×10⁵ particles/µl) was added to 5 ml of the thrombocytopenia model blood, allowed to stand (37°C, 10 min), flowed on a collagen-immobilized surface using a pump (rate of shear 150 s⁻¹), and then observed under a fluorescence microscope or through a CCD camera. An adhesion occupying ratio [surface coverage (hereinafter referred to as "SC")] of micro particles to each surface was measured using an image analyzer (Argus-50, Hamamatsu Photonics).

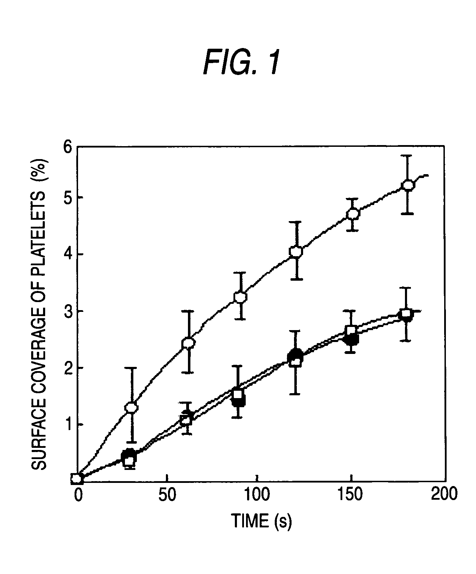

Only platelets were subjected to fluorescence labeling [3,3'-dihexyloxacarbocyanine iodide (DiOC₆)], and their adhesion behavior was periodically observed. As a result, SC of platelets on the surface increased with the lapse of time and was 3.1 ± 0.4% after 180 seconds (Fig. 1: □, control). Also, since this tendency was almost the same as that of a system in which LB was not added, namely the system of platelets alone (3.0 ± 0.6%, Fig. 1: ●), it was suggested that influence upon the platelet adhesion by LB alone does not occur. Thus, when H12-LB was added instead of LB, the platelet adhesion was further amplified than the case of the LB addition, and was 5.1 ± 0.3% after 180 seconds, or amplified about 1.7 times than the control (Fig. 1: o).

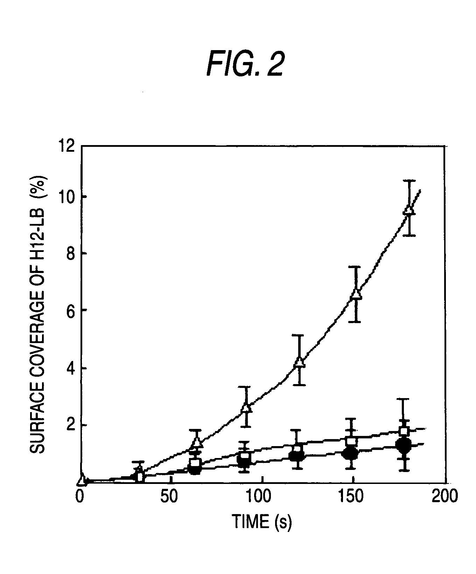

On the other hand, when the adhesion behavior of H12-LB in which LB was labeled with FITC and of LB was observed, initial adhesion rate in the case of H12-LB was markedly gentle in comparison with that of platelets shown in Fig. 1, but the SC was gradually increased (Fig. 2: Δ). In addition, the number of H12-LB bound to the surface was inhibited when the platelet concentration was 0.2×10⁴/µl (Fig. 2: ●), and were hardly seen in the case ofLB (Fig. 2: □).

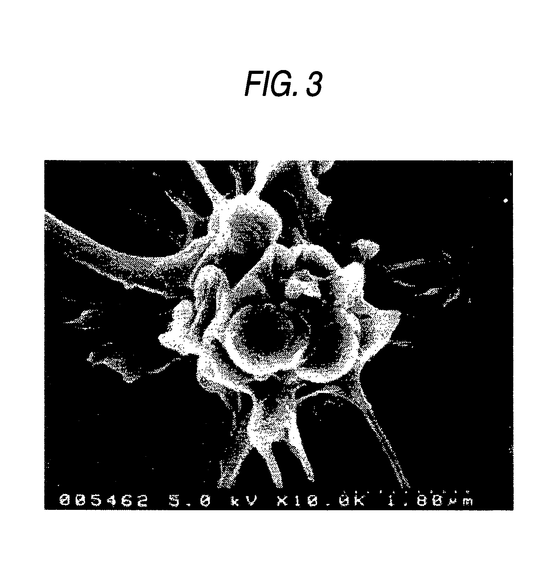

Accordingly, when the surface after flow was observed under SEM (scanning electron microscope), platelets were firstly adhered to the collagen surface and then H12-LB was bound thereto, and further H12-LB-entrained adhesion by other platelets was able to be confirmed (Fig. 3).

Based on the above, it was able to confirm that adhesion of H12-LB to the collagen surface is induced by the presence of platelets, H12-LB is bound on the platelets activated by adhering to collagen, and the flowing platelets are induced and adhered via the same, thereby forming lamination of both in succession.

### Test Example 2

### Binding test to resting platelets:

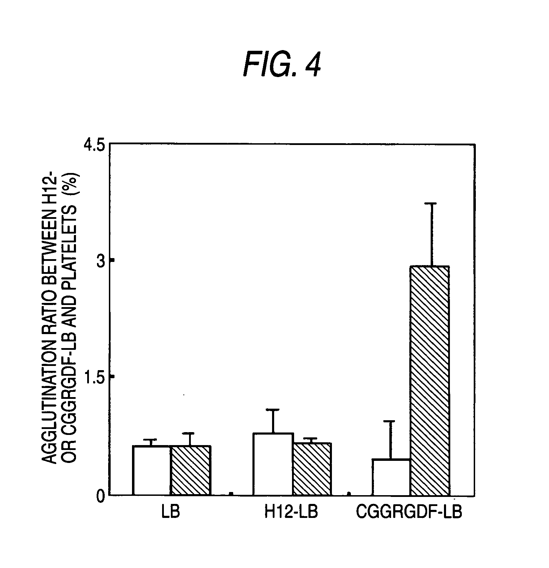

Resting platelets (2.0×10⁴/µl) were mixed with 1.0×10⁵/µl of each of LB, H12-LB and CGGRGDF-LB [LB bound with a peptide having an RGD sequence (Arg-Gly-Asp)], followed by shaking (37°C, 30 min), and the aggregation ratio in each case was evaluated by flow cytometry. As a result, LB and H12-LB did not show increase of the aggregation ratio, but CGGRGDF-LB showed 2.9 + 1.3% (Fig. 4). Based on this, it was confirmed that H12-LB hardly agglutinate to resting platelets when compared with CGGRGDF-LB.

### Test Example 3

### Introduced amount of H12 into vesicles and platelet aggregation effect:

Platelet rich plasma (PRP) was obtained by adding 1/9 (v/v) of an aqueous sodium citrate solution (3.8%) to whole blood, followed by centrifugation (600 rpm, 15 min). Platelet poor plasma (PPP) was obtained by further centrifuging the precipitate (2500 rpm, 10 min). A thrombocytopenia plasma (180 µl, [PLT] = 10×10⁴/µl) was prepared by mixing the PRP and PPP. After 10 µl of a vesicle dispersion was added thereto, 10 µl of ADP (adenosine 5'-phosphate) (300 µM) was further added thereto to thereby aggregate platelets.

Regarding the measurement by aggregometer, increase in the permeability by aggregation when the PPP was regarded as 100% in permeability, and the thrombocytopenia plasma as 0% in permeability, was measure, and a difference in permeability between a case in which H12-PEG(PEG) vesicle was added and a case in which the same concentration of PEG-vesicle was added was evaluated.

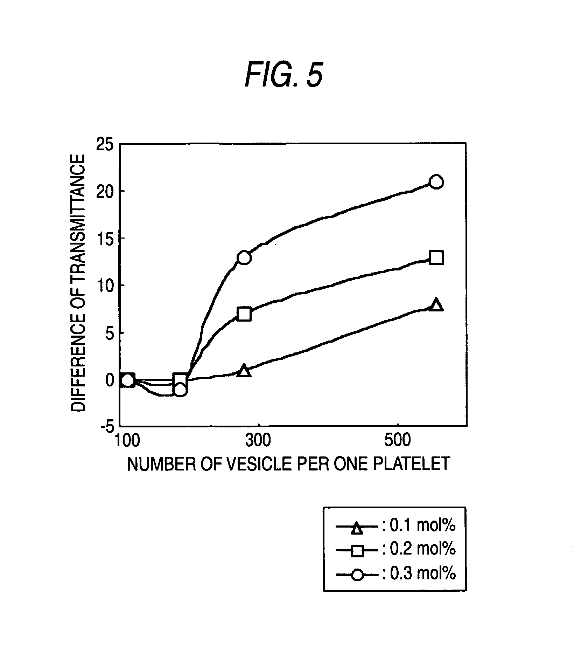

Acceleration of platelet aggregation was found in each of the vesicles containing 0.1 mol%, 0.2 mol% and 0.3 mol% of H12-PEG-Glu2C18, and the aggregation of platelets was accelerated as the amount of H12 introduced into the vesicles increased (Fig. 5).

### Test Example 4

### Aggregation test with platelets when vesicles are used as the carrier:

Platelets were fluorescence-labeled with DiOC₆, and the H12-PEG(PEG) vesicle (1.5 wt%, 200 µl) was added to 5 ml of the thrombocytopenic blood ([platelets] = 5.0×10⁴/µl), allowed to stand (37°C, 10 min), allowed to flow on the collagen surface at a rate of shear of 150 s⁻¹, and then observed under a fluorescence microscope. The SC of platelets on the surface was analyzed by Argus-20. When H12-PEG vesicle (H12-PEG-Glu2C18: 0.3 mol%) or PEG vesicle was added to the thrombocytopenic blood ([platelets] = 5.0× 10⁴/µl) and allowed to flow on the collagen surface, the SC was increased in the H12-PEG vesicle-added system, in comparison with the PEG vesicle-added system (Table 1).

**Table 1**

| Acceleration of platelet aggregation in the presence of H12-PEG(PEG) vesicle or H12-PEG vesicle | |
|---|---|
| Samples | SC of platelets (%) |
| PEG vesicle | 9.6 ± 0.9 |
| H12-PEG vesicle | 15.0 ± 5.0 |
| H12-PEG(PEG) vesicle | 17.3 ± 1.9 |

It is considered that the SC was increased because platelets firstly adhered to the collagen surface, the phospholipid vesicles were bound to the adhered platelets to increase binding sites to the flowing platelets, and platelets were further bound to the phospholipid vesicles. In addition, when the H12-PEG(PEG) vesicles were allowed to flow on the surface under the same conditions, the SC was also increased in the H12-PEG(PEG) vesicle-added system in the same manner, in comparison with the PEG vesicle-added system (Table 1). Thus, it was shown that the recognizing reaction of H12 is not inhibited even when the PEG lipid is introduced on the vesicle surface for the improvement of blood retentivity.

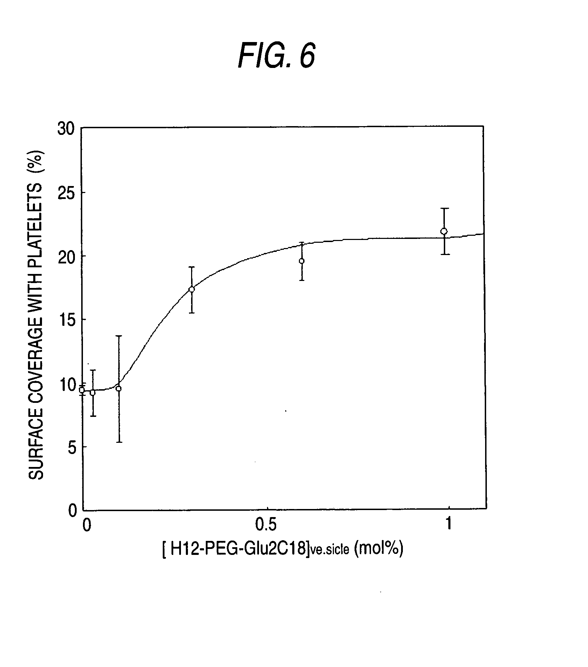

Next, when H12-PEG(PEG) vesicles having different H12-PEG modification ratio were added to the thrombocytopenic blood, the SC of platelets on the collagen surface became as shown in Fig. 6. These results indicate that the SC of platelets on the surface is almost the same as the case of PEG vesicle when the H12-PEG-Glu2C18 modification ratio is 0.1 mol% or less, and the effect appears at a modification ratio of 0.3 mol% or more as the modification ratio increases. The results were considered to be due to the increase of the number of H12-PEG(PEG) vesicles adhered to the platelets activated by collagen, in proportion to the modified amount of H12-PEG-Glu2C18.

### Test Example 5

### Interaction with platelets when vesicles are used as the carrier:

After 80 µl of thrombocytopenia plasma ([platelets] = 5.0×10⁴/µl) was prepared, fluorescence-labeled H12-PEG(PEG) vesicles (8.2 µM, 20 µl) or a fluorescence-labeled antibody and H12-PEG(PEG) vesicles (0.082 µM, 20 µl) were added thereto, followed by stirring (30 min, 37°C). The mixture was subsequently subjected to formaldehyde (3.7%) fixation, diluted 10 times and then measured using flow cytometry.

Regarding the platelets to which H12-PEG(PEG) vesicles were added, as shown in Table 2, the bound amount of the antibody was not changed from the system in which PBS or PEG vesicles were added to platelets. In addition, the H12-PEG(PEG) vesicles themselves hardly showed interaction with platelets, too. Thus, it was confirmed that the H12-PEG(PEG) vesicles do not interact with normal platelets and therefore are not activated in the blood flow. Based on this, it was confirmed that the H12-PEG(PEG) vesicles hardly agglutinate to resting platelets.

**Table 2**

| Binding of PAC-1 to platelets | | |
|---|---|---|
| Samples | PAC-1 binding (%) | vesicle binding (%) |
| PBS | 3.8 ± 0.9 | - |
| PEG vesicles | 3.4 ± 0.3 | 1.3 + 0.7 |
| H12-PEG(PEG) vesicles | 3.7 ± 0.4 | 1.4 + 0.6 |

### Industrial Applicability

Since the conjugate prepared by coupling a synthetic dodecapeptide contained in the GPIIb/IIIa recognizing region of fibrinogen specifically binds only to substantially activated GPIIb/IIIa, it does not induce thrombus formation or intravascular coagulation of blood due to aggregation with resting platelets in blood vessels. In addition, since human-derived components are not used in the peptide conjugate of the present invention, and synthetic forms and recombinant forms of these components are used therein, risk of infection is avoided. Accordingly, the peptide conjugate of the present invention is useful as a platelet substitute.

## Claims

1. A conjugate which is a formula: 
(micro particle)-[(linker)-Cys-His-His-Leu-
Gly-Gly-Ala-Lys-Gln-Ala-Gly-Asp-Val-COOH]n,
 or a formula: 
(micro particle)-[(spacer)-(linker)-Cys-His-His-Leu-
Gly-Gly-Ala-Lys-Gln-Ala-Gly-Asp-Val-COOH]n,
 wherein n is an integer.

2. The conjugate according to claim 1, wherein the micro particle is selected from the group consisting of a vesicle, a micelle, a protein polymer and a synthetic polymer.

3. The conjugate according to claim 1 or 2, wherein the micro particle is selected from the group consisting of a vesicle, a recombinant albumin polymer, a latex particle and a biodegradable polymer.

4. The conjugate according to any one of claims 1 to 3, wherein the micro particle has a particle diameter of 50 nm or more.

5. The conjugate according to any one of claims 1 to 4, wherein the micro particle has a particle diameter of 10 µm or less.

6. The conjugate according to any one of claims 1 to 5, wherein the linker is a compound in which its one terminal reacts with an SH group, and another terminal reacts with any one of an OH group, a COOH group and an NH₂ group.

7. The conjugate according to any one of claims 1 to 6, wherein the linker is selected from the group consisting of dicarboxylic acid, aminocarboxylic acid, a bismaleimide compound, a bishalocarbonyl compound, a halocarbonylmaleimide compound, dithiomaleimide, dithiocarboxylic acid and maleimidecarboxylic acid.

8. The conjugate according to any one of claims 1 to 7, wherein the linker is selected from the group consisting of dicarboxylic acid, aminocarboxylic acid, a bismaleimide compound, a bishalocarbonyl compound, a halocarbonylmaleimide compound, dithiomaleimide, dithiocarboxylic acid and maleimidecarboxylic acid, and has a carbon chain of C2-10.

9. The conjugate according to any one of claims 1 to 8, wherein the spacer is one or at least two combination selected from the group consisting of polyoxyethylene, a polypeptide, a polysaccharide, albumin and an antibody.

10. The conjugate according to any one of claims 1 to 9, wherein the micro particle is a latex particle, the spacer is albumin and the linker is dithiocarboxylic acid.

11. The conjugate according to any one of claims 1 to 9, wherein the micro particle is a vesicle, the spacer is polyethylene glycol and the linker is maleimidecarboxylic acid.

12. A platelet substitute which comprises the conjugate according to any one of claims 1 to 11.

13. A method for controlling platelet aggregation, which comprises using the conjugate according to any one of claims 1 to 11.

14. A process for producing the conjugate according to any one of claims 1 to 11.

15. A diagnostic agent or reagent which comprises the conjugate according to any one of claims 1 to 11.

16. The diagnostic agent or reagent according to claim 15, which is a diagnostic agent for platelet dysfunction, a biological or medical reagent, a platelet aggregation inhibitor, a reagent for screening an anti-thrombus agent, or a diagnostic or therapeutic agent for inspecting a vascular injury region and a thrombus formation region.

17. A drug carrier which comprises the conjugate according to any one of claims 1 to 11.

18. The drug carrier which comprises the conjugate according to claim 17, wherein the drug is selected from the group consisting of a hemostatic agent, a vasoconstrictor agent, an anti-inflammatory agent, an anti-blood coagulation agent and an anti-platelet agent.

19. A pharmaceutical composition which comprises the conjugate according to any one of claims 1 to 11.

20. The pharmaceutical composition which comprises the conjugate according to claim 19, which comprises a drug selected from the group consisting of a hemostatic agent, a vasoconstrictor agent, an anti-inflammatory agent, an anti-blood coagulation agent and an anti-platelet agent.

21. The pharmaceutical composition according to claim 19 or 20, which is useful for preventing or treating vascular disorder, vascular injury and thrombosis.


SEQUENCE LISTING

<110> KEIO UNIVERSITY
<110> Mitsubishi Pharma Corporation

<120> Peptide-conjugated material

<130> GP04-1003

<150> JP P2003-029847
<151> 2003-02-06

<150> JP P2004-017046
<151> 2004-01-26

<160> 2

<170> PatentIn version 3.1

<210> 1
<211> 12
<212> PRT
<213> Artificial

<220>
<223> Designed peptide based on GP IIb/IIIa

<400> 1

His His Leu Gly Gly Ala Lys Gln Ala Gly Asp Val
1               5                   10

<210> 2
<211> 7
<212> PRT
<213> Artificial

<220>
<223> Designed peptide based on GP IIb/IIIa

<400> 1

Cys Gly Gly Arg Gly Asp Phe
1               5